# EUROPÄISCHE PATENTSCHRIFT

(11) **EP 2 945 923 B1**
(45) Veröffentlichungstag und Bekanntmachung des Hinweises auf die Patenterteilung: **15.03.2017**
(21) Anmeldenummer: 14700646.4
(22) Anmeldetag: 15.01.2014
(51) Int. Cl.: C07C 5/48, C07C 7/00, C07C 7/08, C07C 7/11, C07C 7/05, C07C 11/167

(54) **VERFAHREN ZUR OXIDATIVEN DEHYDRIERUNG VON N-BUTENEN ZU BUTADIEN**
METHOD FOR THE OXIDATIVE DEHYDROGENATION OF N-BUTENES TO BUTADIENE
PROCÉDÉ DE DÉSHYDROGÉNATION OXYDATIVE DE N-BUTÈNES EN BUTADIÈNE

(30) Priorität: 15.01.2013 EP 13151362
(43) Veröffentlichungstag der Anmeldung: 25.11.2015
(73) Patentinhaber: BASF SE, 67056 Ludwigshafen am Rhein (DE)
(72) Erfinder: JOSCH, Jan Pablo, 67434 Neustadt (DE); WECK, Alexander, 67251 Freinsheim (DE); GIESA, Sonja, 64293 Darmstadt (DE); BÜTEHORN, Steffen, 68163 Mannheim (DE); BALEGEDDE RAMACHANDRAN, Ragavendra Prasad, 67069 Ludwigshafen (DE); BENFER, Regina, 67122 Altrip (DE); WEBER, Markus, 67117 Limburgerhof (DE)
(86) Internationale Anmeldenummer: PCT/EP2014/050668
(87) Internationale Veröffentlichungsnummer: WO 2014/111406

(56) Entgegenhaltungen:
- WO-A1-2005/063658
- WO-A1-2006/061202
- WO-A1-2014/044693
- WO-A1-2014/086641
- WO-A1-2014/086768
- WO-A1-2014/086813
- WO-A1-2014/086815
- WO-A1-2014/086965
- WO-A2-2011/163053
- US-A- 3 536 775
- US-A- 3 943 185
- US-A1- 2012 130 137
- D. S. ALEXANDER: "Explosions in Butadiene Systems", INDUSTRIAL & ENGINEERING CHEMISTRY, Bd. 51, Nr. 6, 1. Juni 1959 (1959-06-01), Seiten 733-738, XP055112567, ISSN: 0019-7866, DOI: 10.1021/ie50594a026 in der Anmeldung erwähnt

## Beschreibung

Die Erfindung betrifft ein Verfahren zur oxidativen Dehydrierung von n-Butenen zu Butadien.

Butadien ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (AcrylnitrilButadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butanen, Butenen, Butadien, Butinen, Methylallen, C₅-und höheren Kohlenwasserstoffen an.

Butadien kann auch durch oxidative Dehydrierung von n-Butenen (1-Buten und/oder 2-Buten) erhalten werden. Als Ausgangsgasgemisch für die oxidative Dehydrierung von n-Butenen zu Butadien kann jedes beliebige n-Butene enthaltende Gemisch benutzt werden. Beispielsweise kann eine Fraktion verwendet werden, die als Hauptbestandteil n-Butene (1-Buten und/oder 2-Buten) enthält und aus der C₄-Fraktion eines Naphtha-Crackers durch Abtrennen von Butadien und Isobuten erhalten wurde. Des Weiteren können auch Gasgemische als Ausgangsgas eingesetzt werden, die 1-Buten, cis-2-Buten, trans-2-Buten oder deren Gemische umfassen und durch Dimerisierung von Ethylen erhalten wurden. Ferner können als Ausgangsgas n-Butene enthaltende Gasgemische eingesetzt werden, die durch katalytisches Wirbelschichtcracken (Fluid Catalytic Cracking, FCC) erhalten wurden.

n-Butene enthaltende Gasgemische, die als Ausgangsgas in der oxidativen Dehydrierung von n-Butenen zu Butadien eingesetzt werden, können auch durch nicht-oxidative Dehydrierung von n-Butan enthaltenden Gasgemischen hergestellt werden.

WO2005/063658 offenbart ein Verfahren zur Herstellung von Butadien aus n-Butan mit den Schritten
a) Bereitstellung eines n-Butan enthaltenden Einsatzgasstroms a;
b) Einspeisung des n-Butan enthaltenden Einsatzgasstroms a in mindestens eine erste Dehydrierzone und nicht-oxidative katalytische Dehydrierung von n-Butan, wobei ein Produktgasstrom b enthaltend n-Butan, 1-Buten, 2-Buten, Butadien, Wasserstoff, leichtsiedende Nebenbestandteile und gegebenenfalls Wasserdampf erhalten wird;
c) Einspeisung des Produktgasstroms b der nicht-oxidativen katalytischen Dehydrierung und eines sauerstoffhaltigen Gases in mindestens eine zweite Dehydrierzone und oxidative Dehydrierung von 1-Buten und 2-Buten, wobei ein Produktgasstrom c enthaltend n-Butan, 2-Buten, Butadien, Wasserstoff, leichtsiedende Nebenbestandteile und Wasserdampf erhalten wird, welcher einen höheren Gehalt an Butadien als der Produktgasstrom b aufweist;
d) Abtrennung von Wasserstoff, der leichtsiedenden Nebenbestandteile und von Wasserdampf, wobei ein C₄-Produktgasstrom d im Wesentlichen bestehend aus n-Butan, 2-Buten und Butadien erhalten wird;
e) Trennung des C₄-Produktgasstroms d in einen im Wesentlichen aus n-Butan und 2-Buten bestehenden Rückführstrom e1 und einen im Wesentlichen aus Butadien bestehenden Strom e2 durch Extraktivdestillation und Rückführung des Stroms e1 in die erste Dehydrierzone.

Dieses Verfahren zeichnet sich durch eine besonders effektive Ausnutzung der Rohstoffe aus. So werden Verluste des Rohstoffs n-Butan durch Rückführung von nicht umgesetztem n-Butan in die Dehydrierung minimiert. Durch die Koppelung von nichtoxidativer katalytischer Dehydrierung und oxidativer Dehydrierung wird eine hohe Butadien-Ausbeute erzielt. Das Verfahren ist im Vergleich zur Erzeugung von Butadien durch Cracken durch eine hohe Selektivität gekennzeichnet. Es fallen keine Koppelprodukte an. Es entfällt die aufwendige Abtrennung von Butadien aus dem Produktgasgemisch des Crackprozesses.

Der Rest-Sauerstoff kann sich insoweit als störend auswirken, als er in nachgelagerten Verfahrensschritten eine Butadienperoxid-Bildung hervorrufen kann und als Initiator für Polymerisationsreaktionen wirken kann. Diese Gefahr besteht insbesondere in Bereichen, in denen Butadien in hohen Konzentrationen und als Reinstoff vorliegt, da bekannt ist, dass unstabilisiertes 1,3-Butadien in Gegenwart von Sauerstoff gefährliche Butadienperoxide bilden kann. Diese Gefahr besteht in dem oben beschriebenen Prozess inbesondere in Schritt e), in dem Butadien von den restlichen C4-Kohlenwasserstoffen getrennt wird. Diese Risiken wurden zum Beispiel von D. S. Ale-xander (Industrial and Engineering Chemistry 1959, 51, 733 - 738) diskutiert. Diese Peroxide sind viskose Flüssigkeiten. Ihre Dichte ist höher als die von Butadien. Da sie außerdem nur schlecht in flüssigem 1,3-Butadien löslich sind, setzen sie sich auf den Böden von Lagerbehältern ab. Trotz ihrer relativ geringen chemischen Reaktivität sind die Peroxide sehr instabile Verbindungen, die sich bei Temperaturen zwischen 85 und 110 °C spontan zersetzen können. Eine besondere Gefahr besteht in der hohen Schlagempfindlichkeit der Peroxide, die mit der Brisanz eines Sprengstoffes explodieren. Die Gefahr der Polymerbildung ist insbesondere bei der destillativen Abtrennung von Butadien gegeben und kann dort zu Ablagerungen von Polymeren (Bildung von so genanntem "Popcorn") in den Kolonnen führen.
In WO2006/050969 ist ein Verfahren der oxidativen Dehydrierung beschrieben, bei dem eine Sauerstoffentfernung unmittelbar nach der oxidativen Dehydrierung durch eine katalytische Verbrennungsstufe durchgeführt wird. In der katalytischen Verbrennungsstufe wird der Sauerstoff mit in dieser Stufe zugesetztem Wasserstoff in Gegenwart eines Katalysators umgesetzt. Hierdurch wird eine Verringerung des Sauerstoffgehalts bis auf geringe Spuren erreicht. Nachteile der katalytischen Verbrennungsstufe sind der Verbrauch an Wasserstoff sowie Nebenreaktionen, bei der ein Teil des 1,3-Butadiens durch Sauerstoff verbrannt und durch Wasserstoff zu Buten und Butan hydriert wird.

In US 3,943,185 wird die oxidative Dehydrierung von n-Butenen zu Butadien beschrieben. Das dabei entstehende Produktgemisch wird abgekühlt und mit einem hochsiedenden Absorptionsmittel in Kontakt gebracht, wobei ein Gasstrom und ein C4 enthaltender Absorptionsmittelstrom erhalten werden. Der Absorptionsmittelstrom wird in einem Abtrenngefäß dann von Sauerstoff und anderen Gasen befreit. Nachfolgend wird der so erhaltene flüssige Produktstrom durch Strippung vom Absorptionsmittel befreit. Die Schritte C) (Kompression), Db), E) und F) des erfindungsgemässen Verfahrens werden nicht offenbart.

Aufgabe der Erfindung ist es, ein Verfahren der oxidativen Dehydrierung von n-Butenen zu Butadien bereitzustellen, bei dem die Bildung von Butadien-Peroxiden aus Butadien bei der Aufarbeitung des Produktgasgemischs der oxidativen Dehydrierung wirksam verhindert wird.
Gelöst wird die Aufgabe durch ein Verfahren zur Herstellung von Butadien aus n-Butenen mit den folgenden Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, gegebenenfalls Kohlenstoffoxide und gegebenenfalls Inertgase erhalten wird;
C) Abkühlung und Kompression des Produktgasstroms b in mindestens einer Kompressionsstufe, wobei mindestens ein Kondensatstrom c1 enthaltend Wasser und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
D) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch
   Da) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
   Db) Entfernung von Sauerstoff aus dem mit C₄-Kohlenwasserstoffen beladenen Absorptionsmittelstrom durch Strippung mit einem nicht kondensierbaren Gas, und
   Dc) Desorption der C₄-Kohlenwasserstoffe aus dem beladenen Absorptionsmittelstrom, wobei ein C₄-Kohlenwasserstoffstrom d1 erhalten wird, der weniger als *100 ppm* Sauerstoff umfasst;
*E)* Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2.

In einem Schritt A) wird ein n-Butene enthaltender Einsatzgasstrom a bereitgestellt.

Als Einsatzgasstrom a können reine n-Butene (1-Buten und/oder cis-/trans-2-Buten), aber auch Butene enthaltende Gasgemische eingesetzt werden. Ein solches Gasgemisch kann beispielsweise durch nicht-oxidative Dehydrierung von n-Butan erhalten werden. Es kann auch eine Fraktion eingesetzt werden, die als Hauptbestandteil n-Butene (1-Buten und cis-/trans-2-Buten) enthält und aus der C₄-Fraktion des Naphtha-Crackens durch Abtrennung von Butadien und iso-Buten erhalten wurde. Des Weiteren können auch Gasgemische als Ausgangsgas eingesetzt werden, die reines 1-Buten, cis-2-Buten, trans-2-Buten oder Mischungen daraus umfassen, und die durch Dimerisierung von Ethylen erhalten wurden. Ferner können als Ausgangsgas n-Butene enthaltende Gasgemische eingesetzt werden, die durch katalytisches Wirbelschichtkracken (Fluid Catalytic Cracking, FCC) erhalten wurden.

In einer Ausführungsform des erfindungsgemäßen Verfahrens wird das n-Butene enthaltende Ausgangsgasgemisch durch nicht-oxidative Dehydrierung von n-Butan erhalten. Durch die Kopplung einer nicht-oxidativen katalytischen Dehydrierung mit der oxidativen Dehydrierung der gebildeten n-Butene kann eine hohe Ausbeute an Butadien, bezogen auf eingesetztes n-Butan, erhalten werden. Bei der nicht-oxidativen katalytischen n-Butan-Dehydrierung wird ein Gasgemisch erhalten, das neben Butadien, 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

In Schritt B) werden der n-Butene enthaltende Einsatzgasstrom a und ein sauerstoffhaltiges Gas in mindestens eine Dehydrierzone eingespeist und die in dem Gasgemisch enthaltenen Butene in Gegenwart eines Oxidehydrierungskatalysators oxidativ zu Butadien dehydriert.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 (Mo₁₂BiFe_{0,1}Ni₈ZrCr₃K_{0.2}Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A 25 30 959 (Mo₁₂BiFe₃Co_{4,5}Ni_{2,5}Cr_{0,5}K_{0,1}Oₓ, Mo_{13,75}BiFe₃Co_{4,5}Ni_{2,5}Ge_{0,5}K_{0,8}Oₓ, Mo₁₂BiFe₃Co_{4,5}Ni_{2,5}Mn_{0,5}K_{0,1}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ).

Geeignete Multimetalloxide und deren Herstellung sind weiterhin beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Ox), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ).

Besonders bevorzugte katalytisch aktive, Molybdän und mindestens ein weiteres Metall enthaltende Multimetalloxide weisen die allgemeine Formel (la) auf:

M0₁₂BiₐFe_{b}Co_{c}Ni_{d}CrₑX¹_{f}X²_{g}O_{y} (Ia),

mit
X¹ = Si, Mn und/oder Al,
X² = Li, Na, K, Cs und/oder Rb,
0,2 ≤ a ≤ 1,
0,5 ≤ b ≤ 10,
0 ≤ c ≤ 10,
0 ≤ d ≤ 10,
2 ≤ c + d ≤ 10
0 ≤ e ≤ 2,
0 ≤ f ≤ 10
0 ≤ g ≤ 0,5
y = eine Zahl, die unter der Voraussetzung der Ladungsneutralität durch die Wertigkeit und Häufigkeit der von Sauerstoff verschiedenen Elemente in (la) bestimmt wird.

Bevorzugt sind Katalysatoren, deren katalytisch aktive Oxidmasse von den beiden Metallen Co und Ni nur Co aufweist (d = 0). Bevorzugte ist X¹ Si und/oder Mn und X² ist vorzugsweise K, Na und/oder Cs, besonders bevorzugt ist X² = K.

Das molekularen Sauerstoff enthaltende Gas enthält im Allgemeinen mehr als 10 Vol.-%, vorzugsweise mehr als 15 Vol.-% und noch mehr bevorzugt mehr als 20 Vol.-% molekularen Sauerstoff. Bevorzugt ist es Luft. Die Obergrenze für den Gehalt an molekularem Sauerstoff beträgt im Allgemeinen 50 Vol.-% oder weniger, vorzugsweise 30 Vol.-% oder weniger und noch mehr bevorzugt 25 Vol.-% oder weniger. Darüber hinaus können in dem molekularen Sauerstoff enthaltenden Gas beliebige Inertgase enthalten sein. Als mögliche Inertgase können Stickstoff, Argon, Neon, Helium, CO, CO₂ und Wasser genannt werden. Die Menge an Inertgasen beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff beträgt sie im Allgemeinen 10 Vol.-% oder weniger, vorzugsweise 1 Vol.-% oder weniger.

Zur Durchführung der oxidativen Dehydrierung bei Vollumsatz von n-Butenen ist ein Gasgemisch bevorzugt, welches ein molares Sauerstoff: n-Butene-Verhältnis von mindestens 0,5 aufweist. Bevorzugt wird bei einem Sauerstoff: n-Butene-Verhältnis von 0,55 bis 10 gearbeitet. Zur Einstellung dieses Wertes kann das Ausgangsstoffgas mit Sauerstoff oder einem sauerstoffhaltigen Gas, beispielsweise Luft, und gegebenenfalls zusätzlichem Inertgas oder Wasserdampf vermischt werden. Das erhaltene sauerstoffhaltige Gasgemisch wird dann der Oxidehydrierung zugeführt.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z. B. Schmelzen von Salzen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 220 bis 490 °C und bevorzugt zwischen 300 bis 450 °C und besonders bevorzugt zwischen 350 und 420 °C.

Auf Grund der Exothermie der ablaufenden Reaktionen kann die Temperatur in bestimmten Abschnitten des Reaktorinneren während der Reaktion höher liegen als diejenige des Wärmeaustauschmittels und es bildet sich ein so genannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden. Die Differenz zwischen Hotspot-Temperatur und der Temperatur des Wärmeaustauschmittels liegt im Allgemeinen zwischen 1-150 °C, bevorzugt zwischen 10-100 °C und besonders bevorzugt zwischen 20-80 °C. Die Temperatur am Ende des Katalysatorbettes liegt im Allgemeinen zwischen 0-100 °C, vorzugsweise zwischen 0,1-50 °C, besonders bevorzugt zwischen 1-25 °C oberhalb der Temperatur des Wärmeaustauschmittels.

Die Oxidehydrierung kann in allen aus dem Stand der Technik bekannten Festbettreaktoren durchgeführt werden, wie beispielsweise im Hordenofen, im Festbettrohr- oder Rohrbündelreaktor oder im Plattenwärmetauscherreaktor. Ein Rohrbündelreaktor ist bevorzugt.

Vorzugsweise wird die oxidative Dehydrierung in Festbettrohrreaktoren oder Festbettrohrbündelreaktoren durchgeführt. Die Reaktionsrohre werden (ebenso wie die anderen Elemente des Rohrbündelreaktors) in der Regel aus Stahl gefertigt. Die Wanddicke der Reaktionsrohre beträgt typischerweise 1 bis 3 mm. Ihr Innendurchmesser liegt in der Regel (einheitlich) bei 10 bis 50 mm oder bei 15 bis 40 mm, häufig bei 20 bis 30 mm. Die im Rohrbündelreaktor untergebrachte Anzahl an Reaktionsrohren beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Die Länge der Reaktionsrohre erstreckt sich im Normalfall auf wenige Meter, typisch ist eine Reaktionsrohrlänge im Bereich von 1 bis 8 m, häufig 2 bis 7 m, vielfach 2,5 bis 6 m.

Weiterhin kann die Katalysatorschicht, die im Reaktor eingerichtet ist, aus einer einzelnen Schicht oder aus 2 oder mehr Schichten bestehen. Diese Schichten können aus reinem Katalysator bestehen oder mit einem Material verdünnt sein, das nicht mit dem Ausgangsgas oder Komponenten aus dem Produktgas der Reaktion reagiert. Weiterhin können die Katalysatorschichten aus Vollmaterial oder geträgerten Schalenkatalysatoren bestehen.

Der die oxidative Dehydrierung verlassende Produktgasstrom enthält neben Butadien im Allgemeinen noch nicht umgesetztes 1-Buten und 2-Buten, Sauerstoff sowie Wasserdampf. Als Nebenbestandteile enthält er weiterhin im Allgemeinen Kohlenmonoxid, Kohlendioxid, Inertgase (hauptsächlich Stickstoff), leichtsiedende Kohlenwasserstoffe wie Methan, Ethan, Ethen, Propan und Propen, Butan und iso-Butan, gegebenenfalls Wasserstoff sowie gegebenenfalls sauerstoffhaltige Kohlenwasserstoffe, sogenannte Oxygenate.

In Schritt C) wird der Produktgasstrom b abgekühlt und in mindestens einer Kompressionsstufe komprimiert, wobei mindestens ein Kondensatstrom c1 enthaltend Wasser und ein Gasstrom c2 enthaltend Butadien, n-Butene, Sauerstoff, Wasserdampf, leicht siedenden Kohlenwasserstoffe, gegebenenfalls Kohlenstoffoxide und gegebenenfalls Inertgase erhalten wird.

Der Produktgasstrom am Reaktorausgang ist durch eine Temperatur nahe der Temperatur am Ende des Katalysatorbetts charakterisiert. Der Produktgasstrom wird dann auf eine Temperatur von 150 - 400 °C, bevorzugt 160 - 300 °C, besonders bevorzugt 170 - 250 °C gebracht. Es ist möglich, die Leitung, durch die der Produktgasstrom fließt, um die Temperatur im gewünschten Bereich zu halten, zu isolieren, jedoch ist ein Einsatz eines Wärmetauschers bevorzugt. Dieses Wärmetauschersystem ist beliebig, solange mit diesem System die Temperatur des Produktgases auf dem gewünschten Niveau gehalten werden kann. Als Beispiel eines Wärmetauschers können Spiralwärmetauscher, Plattenwärmetauscher, Doppelrohrwärmetauscher, Multirohrwärmetauscher, Kessel-Spiralwärmetauscher, Kessel-Mantelwärmetauscher, Flüssigkeit-Flüssigkeit-Kontakt-Wärmetauscher, Luft-Wärmetauscher, Direktkontaktwärmetauscher sowie Rippenrohrwärmetauscher genannt werden. Da, während die Temperatur des Produktgases auf die gewünschte Temperatur eingestellt wird, ein Teil der hochsiedenden Nebenprodukte, die im Produktgas enthalten sind, ausfallen kann, sollte daher das Wärmetauschersystem vorzugsweise zwei oder mehr Wärmetauscher aufweisen. Falls dabei zwei oder mehr vorgesehene Wärmetauscher parallel angeordnet sind, und so eine verteilte Kühlung des gewonnenen Produktgases in den Wärmetauschern ermöglicht wird, nimmt die Menge an hochsiedenden Nebenprodukten, die sich in den Wärmetauschern ablagern, ab und so kann ihre Betriebsdauer verlängert werden. Als Alternative zu der oben genannten Methode können die zwei oder mehr vorgesehenen Wärmetauscher parallel angeordnet sein. Das Produktgas wird einem oder mehreren, nicht aber allen, Wärmetauschern zugeführt, welche nach einer gewissen Betriebsdauer von anderen Wärmetauschern abgelöst werden. Bei dieser Methode kann die Kühlung fortgesetzt werden, ein Teil der Reaktionswärme zurückgewonnen und parallel dazu können die in einem der Wärmetauscher abgelagerten hochsiedenden Nebenprodukte entfernt werden. Als ein oben genanntes organisches Lösungsmittel kann ein Lösungsmittel, solange es in der Lage ist, die hochsiedenden Nebenprodukte aufzulösen, uneingeschränkt verwendet werden, und als Beispiele dazu können ein aromatisches Kohlenwasserstofflösungsmittel, wie z. B. Toluol, Xylen etc. sowie ein alkalisches wässriges Lösungsmittel, wie z. B. die wässrige Lösung von Natriumhydroxid, verwendet werden.

Anschließend können aus dem Produktgasstrom b durch Abkühlung ein Großteil der hochsiedenden Nebenkomponenten und des Wassers abgetrennt werden. Diese Abkühlung und Abtrennung erfolgt dabei vorzugsweise in einem Quench. Dieser Quench kann aus einer oder mehreren Stufen bestehen. Vorzugsweise wird ein Verfahren eingesetzt, bei dem der Produktgasstrom b direkt mit dem Kühlmedium in Kontakt gebracht und dadurch gekühlt wird. Als Kühlmedium wird vorzugsweise Wasser oder eine alkalische wässrige Lösung verwendet.

Bevorzugt ist ein zweistufiger Quench. Die Kühlungstemperatur des Produktgases unterscheidet sich je nach Art der Temperatur des aus dem Reaktorauslass erhaltenen Produktgases und des Kühlmediums. Im Allgemeinen kann das Produktgas je nach Vorliegen und Temperaturniveau eines Wärmetauschers vor dem Quencheingang eine Temperatur von 100-440 °C, bevorzugt 140-300 °C, insbesondere bevorzugt 170-240 °C erreichen. Der Produktgaseinlass in den Quench muss so ausgelegt sein, dass ein Verstopfen durch Ablagerungen am und direkt vor dem Gaseinlass minimiert oder verhindert wird. Das Produktgas wird in der 1. Quenchstufe mit dem Kühlmedium in Kontakt gebracht. Hierbei kann das Kühlmedium durch eine Düse eingebracht werden, um eine möglichst effiziente Durchmischung mit dem Produktgas zu erreichen. Zum gleichen Zweck können in der Quenchstufe Einbauten, wie zum Beispiel weitere Düsen, eingebracht werden, durch die das Produktgas und das Kühlmedium gemeinsam passieren müssen. Der Kühlmitteleinlass in den Quench muss so ausgelegt sein, dass ein Verstopfen durch Ablagerungen im Bereich des Kühlmitteleinlasses minimiert oder verhindert wird.

Im Allgemeinen wird das Produktgas in der ersten Quenchstufe auf 5-180 °C, vorzugsweise auf 30-130 °C und noch mehr bevorzugt auf 60-90 °C gekühlt. Die Temperatur des Kühlmittelmediums am Einlass kann im Allgemeinen 25-200 °C, bevorzugt 40-120 °C, insbesondere bevorzugt 50-90 °C betragen. Der Druck in der ersten Quenchstufe ist nicht besonders eingeschränkt, beträgt aber im Allgemeinen 0,01-4 bar (ü), bevorzugt 0.1-2 bar (ü) und besonders bevorzugt 0.2-1 bar (ü). Wenn viel hochsiedende Nebenprodukte im Produktgas vorhanden sind, kommt es leicht zu Polymerisation unter den hochsiedenden Nebenprodukten oder zu Ablagerungen von festen Nebenprodukten, die durch hochsiedende Nebenprodukte in diesem Arbeitsschritt verursacht werden. Das im Kühlturm eingesetzt Kühlmedium wird häufig zirkulierend eingesetzt, sodass es zu Blockaden durch feste Niederschläge kommen kann, wenn die Herstellung von konjugierten Dienen kontinuierlich fortgesetzt wird. Der Kreislaufstrom des Kühlmediums in Liter pro Stunde bezogen auf den Massenstrom an Butadien in Gramm pro Stunde kann im Allgemeinen 0.0001-5 l/g, bevorzugt 0.001-1 l/g und besonders bevorzugt 0.002-0.2 l/g betragen.

Das Einlösen von Nebenprodukten der ODH-Reaktion, zum Beispiel von Essigsäure, MSA, etc. in ein Kühlmedium wie zum Beispiel Wasser gelingt bei erhöhtem pH-Wert besser als bei niedrigem pH-Wert. Da das Einlösen von Nebenprodukten wie den oben genannten pH-Wert von zum Beispiel Wasser erniedrigt, kann der pH-Wert durch Zugabe eines alkalischen Mediums konstant gehalten oder erhöht werden. Im Allgemeinen wird der pH-Wert im Sumpf der ersten Quenchstufe zwischen 2-14, bevorzugt zwischen 3-13, besonders bevorzugt zwischen 4-12 gehalten. Je saurer der Wert, desto weniger alkalisches Medium muss zugeführt werden. Je basischer, desto besser gelingt die Einlösung einiger Nebenprodukte. Jedoch führen sehr hohe pH-Werte zum Einlösen von Nebenprodukten wie CO₂ und damit zu einem sehr hohen Verbrauch des alkalischen Mediums. Die Temperatur des Kühlmediums im Sumpf kann im Allgemeinen 27-210 °C, bevorzugt 45-130 °C, insbesondere bevorzugt 55-95 °C betragen. Da die Beladung des Kühlmediums mit Nebenkomponenten im Laufe der Zeit zunimmt, kann ein Teil des beladenen Kühlmediums aus dem Umlauf abgezogen werden und die Umlaufmenge durch Zugabe von unbeladenem Kühlmedium konstant gehalten werden. Das Verhältnis von Ablaufmenge und Zugabemenge hängt von der Dampfbeladung des Produktgases und der Produktgastemperatur am Ende der erste Quenchstufe ab. Wenn das Kühlmedium Wasser ist, ist die Zugabemenge in der ersten Quenchstufe im Allgemeinen geringer als die Ablaufmenge.

Der abgekühlte und an Nebenkomponenten abgereicherte Produktgasstrom kann nun einer zweiten Quenchstufe zugeführt werden. In dieser kann er nun erneut mit einem Kühlmedium in Kontakt gebracht werden.

Im Allgemeinen wird das Produktgas bis zum Gasausgang der zweiten Quenchstufe auf 5 bis 100 °C, vorzugsweise auf 15-85 °C und noch mehr bevorzugt auf 30-70 °C gekühlt. Das Kühlmittel kann im Gegenstrom zum Produktgas zugeführt werden. In diesem Fall kann die Temperatur des Kühlmittelmediums am Kühlmitteleinlass 5-100 °C, bevorzugt 15-85 °C, insbesondere bevorzugt 30-70 °C betragen. Der Druck in der zweiten Quenchstufe ist nicht besonders eingeschränkt, beträgt aber im Allgemeinen 0,01-4 bar (ü), bevorzugt 0,1-2 bar (ü) und besonders bevorzugt 0,2-1 bar (ü). Das im Kühlturm eingesetzte Kühlmedium wird häufig zirkulierend eingesetzt, so dass es zu Blockaden durch feste Niederschläge kommen kann, wenn die Herstellung von konjugierten Dienen kontinuierlich fortgesetzt wird. Der Kreislaufstrom des Kühlmediums in Liter pro Stunde, bezogen auf den Massenstrom an Butadien in Gramm pro Stunde, kann im Allgemeinen 0,0001-5 l/g, bevorzugt 0,001-1 l/g und besonders bevorzugt 0,002-0,2 l/g betragen.

Das Einlösen von Nebenprodukten der ODH-Reaktion, zum Beispiel von Essigsäure, MSA, etc. in einem Kühlmedium wie zum Beispiel Wasser gelingt bei erhöhtem pH-Wert besser als bei niedrigem pH-Wert. Da das Einlösen von Nebenprodukten wie den oben genannten pH-Wert von zum Beispiel Wasser erniedrigt, kann der pH-Wert durch Zugabe eines alkalischen Mediums konstant gehalten oder erhöht werden. Im Allgemeinen wird der pH-Wert im Sumpf der zweiten Quenchstufe zwischen 1-14, bevorzugt zwischen 2-12, besonders bevorzugt zwischen 3-11 gehalten. Je saurer der Wert, desto weniger alkalisches Medium muss zugeführt werden. Je basischer, desto besser gelingt die Einlösung einiger Nebenprodukte. Jedoch führen sehr hohe pH-Werte zum Einlösen von Nebenprodukten wie CO₂ und damit zu einem sehr hohen Verbrauch des alkalischen Mediums. Die Temperatur des Kühlmediums im Sumpf kann im Allgemeinen 20-210 °C, bevorzugt 35-120 °C, insbesondere bevorzugt 45-85 °C betragen. Da die Beladung des Kühlmediums mit Nebenkomponenten im Laufe der Zeit zunimmt, kann ein Teil des beladenen Kühlmediums aus dem Umlauf abgezogen werden und die Umlaufmenge durch Zugabe von unbeladenem Kühlmedium konstant gehalten werden. Das Verhältnis von Ablaufmenge und Zugabemenge hängt von der Dampfbeladung des Produktgases und der Produktgastemperatur am Ende der zweiten Quenchstufe ab. Wenn das Kühlmedium Wasser ist, ist die Zugabemenge in der zweiten Quenchstufe im Allgemeinen größer als die Ablaufmenge.

Um einen möglichst guten Kontakt von Produktgas und Kühlmedium zu erreichen, können Einbauten in der zweiten Quenchstufe vorhanden sein. Solche Einbauten umfassen zum Beispiel Glocken-, Zentrifugal- und/oder Siebböden, Kolonnen mit strukturierten Packungen, z. B. Blechpackungen mit einer spezifischen Oberfläche von 100 bis 1000 m2/m3 wie Mellapak® 250 Y, und Füllkörperkolonnen.

Die Umläufe der beiden Quenchstufen können sowohl voneinander getrennt als auch miteinander verbunden sein. Die gewünschte Temperatur der Umlaufströme kann über geeignete Wärmetauscher eingestellt werden.

Um den Mitriss von flüssigen Bestandteilen aus dem Quench in die Abgasleitung zu minimieren, können geeignete bauliche Maßnahmen, wie zum Beispiel der Einbau eines Demisters, getroffen werden. Weiterhin können hochsiedende Substanzen, welche im Quench nicht vom Produktgas abgetrennt werden, durch weitere bauliche Maßnahmen, wie beispielsweise Gaswäschen, aus dem Produktgas entfernt werden. Es wird ein Gasstrom erhalten, in welchem n-Butan, 1-Buten, 2-Butene, Butadien, gegebenenfalls Sauerstoff, Wasserstoff, Wasserdampf, in geringen Mengen Methan, Ethan, Ethen, Propan und Propen, iso-Butan, Kohlenstoffoxide und Inertgase verbleibt. Weiterhin können in diesem Produktgasstrom Spuren von hochsiedenden Komponenten verbleiben, welche im Quench nicht quantitativ abgetrennt wurden.

Anschließend wird in Schritt C) der Produktgasstrom b aus dem Quench in mindestens einer Kompressionsstufe komprimiert und nachfolgend weiter abgekühlt, wobei mindestens ein Kondensatstrom c1 enthaltend Wasser auskondensiert und ein Gasstrom c2 enthaltend Butadien, 1-Buten, 2-Butene, Sauerstoff, Wasserdampf, gegebenenfalls leichtsiedende Kohlenwasserstoffe wie Methan, Ethan, Ethen, Propan und Propen, Butan und iso-Butan, gegebenenfalls Kohlenstoffoxide und gegebenenfalls Inertgase verbleibt.

Die Kompression kann ein- oder mehrstufig erfolgen. Im Allgemeinen wird insgesamt von einem Druck im Bereich von 1,0 bis 4,0 bar (absolut) auf einen Druck im Bereich von 3,5 bis 20 bar (absolut) komprimiert. Nach jeder Kompressionsstufe folgt eine Abkühlstufe, in der der Gasstrom auf eine Temperatur im Bereich von 15 bis 60 °C abgekühlt wird. Der Kondensatstrom kann somit bei mehrstufiger Kompression auch mehrere Ströme umfassen. Der Kondensatstrom besteht im Allgemeinen zu mindestens 80 Gew.-%, vorzugsweise zu mindestens 90 Gew.-% aus Wasser und enthält daneben in geringem Umfang Leichtsieder, C₄-Kohlenwasserstoffe, Oxygenate und Kohlenstoffoxide.

Der Butadien, n-Butene, Sauerstoff, leicht siedende Kohlenwasserstoffe (Methan, Ethan, Ethen, Propan, Propen, n-Butan, iso-Butan), gegebenenfalls Wasserdampf, gegebenenfalls Kohlenstoffoxide sowie gegebenenfalls Inertgase enthaltende Gasstrom c2 wird als Ausgangsstrom der weiteren Aufbereitung zugeführt.

In einem Schritt D) werden nicht kondensierbare und leicht siedende Gasbestandteile, umfassend Sauerstoff, leicht siedenden Kohlenwasserstoffe (Methan, Ethan, Ethen, Propan, Propen), Kohlenstoffoxide und Inertgase als Gasstrom d2 aus dem Gasstrom c2 durch Absorption der C₄-Kohlenwasserstoffe in einem hochsiedenden Absorptionsmittel und nachfolgender Desorption der C₄-Kohlenwasserstoffe abgetrennt durch
Da) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
Db) Entfernung von Sauerstoff aus dem mit C₄-Kohlenwasserstoffen beladenen Absorptionsmittelstrom durch Strippung mit einem Inertgas, und
Dc) Desorption der C₄-Kohlenwasserstoffe aus dem beladenen Absorptionsmittelstrom, wobei ein C₄-Produktgasstrom d1 erhalten wird, der im Wesentlichen aus C₄-Kohlenwasserstoffen besteht und weniger als *100 ppm* Sauerstoff umfasst.

Bevorzugt enthält der C₄-Produktgasstrom weniger als 50 ppm, besonders bevorzugt weniger als 20 ppm und insbesondere bevorzugt weniger als 10 ppm Sauerstoff.

Dazu wird in der Absorptionsstufe Da) der Produktgasstrom nach der Wasserabtrennung erhaltene Gasstrom c2 mit einem inerten Absorptionsmittel in Kontakt gebracht und werden die C₄-Kohlenwasserstoffe in dem inerten Absorptionsmittel absorbiert, wobei ein mit C₄-Kohlenwasserstoffen beladenes Absorptionsmittel und ein die übrigen Gasbestandteile enthaltendes Abgas erhalten werden. In einer Desorptionsstufe werden die C₄-Kohlenwasserstoffe aus dem hochsiedenden Absorptionsmittel wieder freigesetzt.

Die Absorptionsstufe kann in jeder beliebigen, dem Fachmann bekannten geeigneten Absorptionskolonne durchgeführt werden. Die Absorption kann durch einfaches Durchleiten des Produktgasstroms durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme, Graphitblockabsorber, Oberflächenabsorber wie Dickschicht und Dünnschichtabsorber sowie Rotationskolonnen, Tellerwäscher, Kreuzschleierwäscher und Rotationswäscher in Betracht.

In einer Ausführungsform wird einer Absorptionskolonne im unteren Bereich der Butadien, n-Butene und die leichtsiedenden und nicht kondensierbaren Gasbestandteile enthaltende Gasstrom c2 zugeführt. Im oberen Bereich der Absorptionskolonne wird das hochsiedende Absorptionsmittel aufgegeben.

In der Absorptionsstufe eingesetzte inerte Absorptionsmittel sind im Allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Geeignete Absorptionsmittel sind vergleichsweise unpolare organische Lösungsmittel, beispielsweise aliphatische C₈- bis C₁₈-Alkane, oder aromatische Kohlenwasserstoffe wie die Mittelölfraktionen aus der Paraffindestillation, Toluol oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%.

Bevorzugte Absorptionsmittel sind Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

In einer bevorzugten Ausführungsform wird als Lösungsmittel für die Absorption ein Alkangemisch wie Tetradekan (technischer C14-C17 Schnitt) eingesetzt.

Am Kopf der Absorptionskolonne wird ein Abgasstrom abgezogen, der im Wesentlichen Sauerstoff, leicht siedende Kohlenwasserstoffe (Methan, Ethan, Ethen, Propan, Propen), gegebenenfalls C₄-Kohlenwasserstoffe (Butan, Butene, Butadien), gegebenenfalls Inertgase, gegebenenfalls Kohlenstoffoxide und gegebenenfalls noch Wasserdampf enthält. Dieser Stoffstrom kann teilweise dem ODH-Reaktor zugeführt werden. Damit lässt sich zum Beispiel der Eintrittsstrom des ODH-Reaktors auf den gewünschten C₄-Kohlenwasserstoffgehalt einstellen.

Am Sumpf der Absorptionskolonne werden in einer weiteren Kolonne durch die Spülung mit einem Gas Reste von im Absorptionsmittel gelöstem Sauerstoff ausgetragen. Der verbleibende Sauerstoffanteil soll so klein sein, dass der die Desorptionskolonne verlassende und Butan, Buten sowie Butadien enthaltende Strom d1 nur noch maximal 100 ppm Sauerstoff enthält.

Das Ausstrippen des Sauerstoffs kann in jeder beliebigen, dem Fachmann bekannten geeigneten Kolonne durchgeführt werden. Das Strippen kann durch einfaches Durchleiten von nicht kondensierbaren Gasen, bevorzugt einem Inertgas wie Stickstoff, durch die beladene Absorptionslösung erfolgen. Mit ausgestripptes C4 wird zurück in die Absorptionslösung gewaschen, indem der Gasstrom durch die Absorptionskolonne zurück geleitet wird. Das kann in einer bevorzugten Ausführungsform durch direkte Montage der Stripperkolonne unterhalb der Absorberkolonne erfolgen. Da der Druck im Strippkolonnenteil und Absorptionskolonnenteil erfindungsgemäß gleich ist, kann diese direkte Kopplung erfolgen. Geeignete Stippkolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme sowie Rotationskolonnen, Tellerwäscher, Kreuzschleierwäscher und Rotationswäscher in Betracht. Geeignete Gase sind zum Beispiel Stickstoff oder Methan.

Der mit C₄-Kohlenwasserstoffen beladene Absorptionsmittelstrom wird anschließend in eine Desorptionskolonne geleitet. In einer Verfahrensvariante wird der Desorptionsschritt Dc) durch Entspannung und/oder Erhitzen des beladenen Absorptionsmittels durchgeführt. Bevorzugte Verfahrensvariante ist die Zugabe von Strippdampf und/oder die Zufuhr von Frischdampf im Sumpf der Desorptionskolonne. Das von C₄-Kohlenwasserstoffen abgereicherte Absorptionsmittel kann als Gemisch gemeinsam mit dem kondensierten Wasserdampf einer Phasentrennung zugeführt werden.

Bevorzugt enthält das Absorptionsmittel nach dem Strippen mit Wasserdampf 70 bis 100 Gew.-% hochsiedendes Lösungsmittel und 0 bis 30 Gew.-% Wasser, besonders bevorzugt 80 bis 100 Gew.-% hochsiedendes Lösungsmittel und 0 bis 20 Gew.-% Wasser, insbesondere 85 bis 95 Gew.-% hochsiedendes Lösungsmittel und 5 bis 15 Gew.-% Wasser. Das in der Desorptionsstufe regenerierte Absorptionsmittel wird in die Absorptionsstufe zurückgeführt.

Der im Wesentlichen aus n-Butan, n-Butenen und Butadien bestehende C₄-Produktgasstrom d1 enthält im Allgemeinen 20 bis 80 Vol.-% Butadien, 0 bis 80 Vol.-% n-Butan, 0 bis 10 Vol.-% 1-Buten und 0 bis 50 Vol.-% 2-Butene, wobei die Gesamtmenge 100 Vol.-% ergibt. Weiterhin können geringe Mengen an Iso-Butan enthalten sein.

Der C4-Produktstrom d1 wird anschließend durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2 aufgetrennt.

Der im Wesentlichen aus n-Butan und 2-Buten bestehende Strom f2 kann ganz oder teilweise dem C₄-Feed des ODH-Reaktors zugeführt werden. Da die Buten-Isomere dieses Rückführstroms im Wesentlichen aus 2-Butenen bestehen, und 2-Butene im Allgemeinen langsamer zu Butadien oxidativ dehydriert werden als 1-Buten, kann dieser Rückführstrom vor der Zuführung in den ODH-Reaktor katalytisch isomerisiert werden. Dadurch kann die Isomerenverteilung entsprechend der im thermodynamischen Gleichgewicht vorliegenden Isomerenverteilung eingestellt werden.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der C₄-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu C₄-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250 °C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte zyklische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte zyklische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmitteln und/oder tert.-Butylether, z.B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

Der Kopfproduktstrom der Extraktivdestillationskolonne enthält im Wesentlichen Butan und Butene und in geringen Mengen Butadien und wird gasförmig oder flüssig abgezogen. Im Allgemeinen enthält der im Wesentlichen aus n-Butan und 2-Buten bestehende Strom 50 bis 100 Vol-% n-Butan, 0 bis 50 Vol-% 2-Buten und 0 bis 3 Vol-% weitere Bestandteile wie iso-Butan, Isobuten, Propan, Propen und C₅⁺-Kohlenwasserstoffe.

In einem Schritt F) wird der Butadien und das selektive Lösungsmittel enthaltende Stoffstrom e1 in einen im Wesentlichen aus dem selektiven Lösungsmittel bestehenden Stoffstrom f1 und einen Butadien enthaltenden Stoffstrom f2 *destillativ* aufgetrennt.

Der am Sumpf der Extraktivdestillationskolonne gewonnene Stoffstrom e1 enthält im Allgemeinen das Extraktionsmittel, Wasser, Butadien und in geringen Anteilen Butene und Butan und wird einer *Destillationskolonne* zugeführt. In dieser kann über Kopf Butadien gewonnen werden. Am Sumpf der Destillationskolonne fällt ein Extraktionsmittel und gegebenenfalls Wasser enthaltender Stoffstrom an, wobei die Zusammensetzung des Extraktionsmittel und Wasser enthaltenden Stoffstroms der Zusammensetzung entspricht, wie sie der Extraktion zugegeben wird. Der Extraktionsmittel und Wasser enthaltende Stoffstrom wird bevorzugt in die Extraktivdestillation zurückgeleitet.

In einer Variante wird in der Destillationskolonne ein Butadien und das Extraktionsmittel enthaltender Seitenabzugsstrom gewonnen und in eine Desorptionszone überführt. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion aus Butadien zuvor kondensiert wird. Als Einbauten sind Packungen, Böden oder Füllkörper vorgesehen. Die Destillation wird vorzugsweise bei einer Sumpftemperatur im Bereich von 100 bis 300 °C, insbesondere im Bereich von 150 bis 200 °C und einer Kopftemperatur im Bereich von 0 bis 70 °C, insbesondere im Bereich von 10 bis 50 °C durchgeführt. Der Druck in der Destillationskolonne liegt dabei vorzugsweise im Bereich von 1 bis 10 bar. Im Allgemeinen herrschen in der Desorptionszone gegenüber der Extraktionszone ein verminderter Druck und/oder eine erhöhte Temperatur.

Der am Kolonnenkopf gewonnene Wertproduktstrom enthält im Allgemeinen 90 bis 100 Vol.-% Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

Die Erfindung wird durch die nachstehenden Beispiele näher erläutert.

### Beispiele

### Beispiel 1:

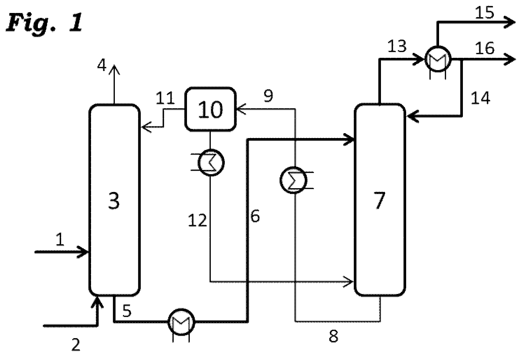

Eine Ausführungsform des erfindungsgemäßen Verfahrens ist in Figur 1 dargestellt. Darin bedeuten die Bezeichnungen:
1: Prozessgasstrom mit O₂, N₂, C₄-Kohlenwasserstoffe, CO, CO₂, H₂O
2: Strippmedium, nichtkondensierbares Gas
5: mit C₄-Kohlenwasserstoffen beladene Absorptionslösung, kühl
4: Abgasstrom mit O₂, N₂, CO, CO₂
6: mit C₄-Kohlenwasserstoffen beladene Absorptionslösung, erwärmt
8: unbeladene Absorptionslösung mit H₂O
11: unbeladene Absorptionslösung
12: unbeladenes Wasser
13: C₄-Kohlenwasserstoffstrom, gasförmig
14: C₄-Kohlenwasserstoffstrom, flüssiger Rücklauf
15: gasförmiger C₄-Kohlenwasserstoffstrom zur Extraktivdestillation, enthält noch Inertgase
16: kondensierter C₄-Kohlenwasserstoffstrom zur Extraktivdestillation

Das den Kompressor verlassende Prozessgasgemisch tritt mit 40°C und der in Tabelle 1 gezeigten Zusammensetzung als Strom 1 auf der 30. Stufe der 60 Stufen umfassenden Absorptionskolonne in diese ein. Der Kopfdruck der Kolonne beträgt 10 bar absolut und hat Glockenböden.

**Tabelle 1**

| Komponente | Gewichts-% |
|---|---|
| BUTAN | 3,82 |
| I-BUTAN | 0,42 |
| I-BUTEN | 0,03 |
| 1-BUTEN | 0,01 |
| C-2-BUTEN | 0,35 |
| T-2-BUTEN | 1,04 |
| 1.3-BUTADIEN | 14,47 |
| H₂O | 0,42 |
| TETRADECAN | 0,00 |
| CO2 | 1,58 |
| N2 | 70,44 |
| 02 | 7,43 |

In der Absorberkolonne strömt der Prozessgasstrom **1** im Gegenstrom zum von oben zugeführten Absorbensstrom **11** bestehend hauptsächlich aus mit Wasser gesättigtem Tetradecan. Dabei nimmt das Absorbens bevorzugt die C₄-Kohlenwasserstoffe und geringe Anteile der nicht kondenstierbaren Gasen auf. Das Verhältnis zwischen der Masse des Absorbensstroms **11** und des Prozessgasstroms **1** liegt bei 2,9 zu 1. Der untere Teil der Kolonne wird als Strippkolonne genutzt, und dem beladenen Absorbensstrom wird von der untersten Stufe der Kolonne Stickstoff (Strom **2**) mit einer Temperatur von 35°C entgegen geführt. Das Massenverhältnis zwischen Absorbensstrom und Stickstoffstrom **2** beträgt 98 zu 1. Durch dieses Strippen wird der Sauerstoff aus dem mit C₄-Komponenten beladenen Absorbensstrom entfernt und der Strom **5** tritt mit 46,6°C und der in Tabelle 2 gezeigten Zusammensetzung aus der Absorptionskolonne aus.

**Tabelle 2**

| Komponente | Gewichts-% |
|---|---|
| BUTAN | 1,24 |
| I-BUTAN | 0,13 |
| I-BUTEN | 0,01 |
| 1-BUTEN | 0,00 |
| C-2-BUTEN | 0,11 |
| T-2-BUTEN | 0,34 |
| 1.3-BUTADIEN | 4,66 |
| H2O | 0,05 |
| TETRADECAN | 93,37 |
| CO2 | 0,00 |
| N2 | 0,08 |
| O2 | 0,019 ppm |

Die nicht kondensierbaren Gase verlassen die Absorptionskolonne hauptsächlich als Strom **4** über den Kopf der Kolonne mit einer Temperatur von 35°C und der in Tabelle 3 gezeigten Zusammensetzung.

**Tabelle 3**

| Komponente | Gewichts-% |
|---|---|
| BUTAN | 0,01 |
| I-BUTAN | 0,03 |
| I-BUTEN | 0,00 |
| 1-BUTEN | 0,00 |
| C-2-BUTEN | 0,00 |
| T-2-BUTEN | 0,00 |
| 1.3-BUTADIEN | 0,18 |
| H2O | 0,36 |
| TETRADECAN | 0,00 |
| CO2 | 1,89 |
| N2 | 88,53 |
| O2 | 9,00 |

Der Absorptionsmittelstrom **5** wird auf 50°C erhitzt und tritt als Strom **6** auf der 30. Stufe der 36 Glockenböden umfassenden Desorptionskolonne in diese ein. Die Desorptionskolonne wird mit einem Kopfdruck von 5,5 bar absolut betrieben und es werden in ihr die C₄-Kohlenwasserstoffe mit dem 156°C heißen Wasserdampfstrom **12** aus der Absorptionsmischung gestrippt. Das Gemisch aus Wasser und nicht mehr beladenem Absorptionsmittel verlässt den Sumpf der Desorptionskolonne als Strom **8** mit einer Temperatur von 155°C und besitzt die Zusammensetzung wie in Tabelle 4 aufgeführt.

**Tabelle 4**

| Komponente | Gewichts-% |
|---|---|
| BUTAN | 0,00 |
| I-BUTAN | 0,00 |
| I-BUTEN | 0,00 |
| 1-BUTEN | 0,00 |
| C-2-BUTEN | 0,00 |
| T-2-BUTEN | 0,00 |
| 1.3-BUTADIEN | 0,00 |
| H2O | 10,74 |
| TETRADECAN | 89,26 |
| CO2 | 0,00 |
| N2 | 0,00 |
| O2 | 0,00 |

Der Strom **8** wird anschließend auf 35°C abgekühlt, auf 10 bar absolut komprimiert und im Dekanter **10** in einen Tetradekan-reichen Strom **11** und einen wässrigen Strom **12** getrennt.

Am Kopf der Desorptionskolonne werden die C₄-Komponenten im Strom **13** bei einer Temperatur von 48°C und der in Tabelle 5 gezeigten Zusammensetzung abgezogen, anschließend auf 15°C abgekühlt und damit teilweise kondensiert.

**Tabelle 5**

| Komponente | Gewichts-% |
|---|---|
| BUTAN | 18,86 |
| I-BUTAN | 1,93 |
| I-BUTEN | 0,13 |
| 1-BUTEN | 0,05 |
| C-2-BUTEN | 1,72 |
| T-2-BUTEN | 5,16 |
| 1.3-BUTADIEN | 70,80 |
| H2O | 0,67 |
| TETRADECAN | 0,00 |
| CO2 | 0,04 |
| N2 | 0,63 |
| O2 | 0,00 |

Das Kondensat wird zum Teil in den Kopf der Desorptionskolonne zurückgeführt. Das Massenverhältnis zwischen den Strömen **14** und **15** ist hier 1 zu 1. Die Zusammensetzung des gasförmigen Stroms **15** und des flüssigen Stroms **16** sind in Tabelle 6 aufgeführt.

**Tabelle 6**

| Komponente | Strom 15 Gewichts-% | Strom 16 Gewichts-% |
|---|---|---|
| BUTAN | 9,92 | 18,99 |
| I-BUTAN | 1,54 | 1,94 |
| I-BUTEN | 0,07 | 0,13 |
| 1-BUTEN | 0,03 | 0,05 |
| C-2-BUTEN | 0,69 | 1,73 |
| T-2-BUTEN | 2,25 | 5,20 |
| 1.3-BUTADIEN | 38,66 | 71,26 |
| H2O | 0,15 | 0,68 |
| TETRADECAN | 0,00 | 0,00 |
| CO2 | 2,36 | 0,01 |
| N2 | 44,33 | 0,00 |
| O2 | 10 ppm | 0,00 |

Bei diesen Betriebsbedingungen wird eine Sauerstoffkonzentration von 10 ppm im Gasstrom hinter der C₄-Absorption/Desorption erreicht.

### Beispiel 2:

Eine weitere Variante des erfindungsgemäßen Gedanken ist in Figur 1 dargestellt. Bezugnehmend auf Figur 1 darin bedeuten die Bezeichnungen:
1: Prozessgasstrom mit O2, N2, C4, H2O
2: Strippmedium, nichtkondensierbares Gas
5: mit C4 beladene Absorptionslösung, kühl
4: Abgasstrom mit O2, N2
6: mit C4 beladene Absorptionslösung, erwärmt
8: unbeladene Absorptionslösung mit H2O
11: unbeladene Absorptionslösung
12: unbeladenes Wasser
13: C4-Strom, gasförmig
14: C4-Strom, flüssiger Rücklauf
16: kondensierter C4-Strom zur Extraktivdestillation
15: gasförmiger C4-Strom zur Extraktivdestillation, enthält noch Inertgase

Das den Kompressor verlassende Prozessgasgemisch tritt mit 41 °C und der in Tabelle 7 gezeigten Zusammensetzung als Strom **1** auf der 10. realen Stufe der 40 reale Stufen umfassenden Absorptionskolonne in diese ein. Der Kopfdruck der Kolonne beträgt 10 bar absolut und hat Glockenböden. Die Bestimmung der in den folgenden Tabellen angebenden Volumenanteile erfolgte gaschromatographisch. Die Kalibrierung erfolgt hierbei mittels eines externen Standards.

**Tabelle 7**

| Komponente | Vol.-% |
|---|---|
| BUTAN | 2,6 |
| I-BUTAN | 0,8 |
| I-BUTEN | 0,02 |
| 1-BUTEN | 0,04 |
| C-2-BUTEN | 0,2 |
| T-2-BUTEN | 0,4 |
| 1.3-BUTADIEN | 10,7 |
| H2O | 0,06 |
| Sauerstoff | 5,4 |
| Stickstoff | 79,78 |

In der Absorberkolonne strömt der Prozessgasstrom im Gegenstrom zum von oben zugeführten Absorbensstrom **11**, der hauptsächlich aus mit Wasser gesättigtem Mestiylen (1,3,4-trimethylbenzol) besteht. Dabei nimmt das Absorbens bevorzugt die C4-Kohlenwasserstoffe auf und geringe Anteile der nicht kondenstierbaren Gasen auf. Das Verhältnis zwischen der Masse des Absorbensstroms **11** und des Prozessgasstroms **1** liegt bei 2,9 zu 1. Der untere Teil der Kolonne wird als Strippkolonne genutzt und dem beladenen Absorbenstrom von der untersten Stufe der Kolonne Stickstoff (Strom **2**) mit einer Temperatur von 35°C entgegen geführt. Das Massenverhältnis zwischen beladenem Absorbensstrom und Stickstoffstrom **2** beträgt 328 zu 1. Durch dieses Strippen wird der Sauerstoff aus dem mit C₄-Komponenten beladenen Absorbensstrom entfernt und der Strom **5** tritt mit 43°C aus der Absorptionskolonne aus.

Die nicht kondensierbaren Gase verlassen die Absorptionskolonne hauptsächlich als Strom **4** über den Kopf der Kolonne mit einer Temperatur von 35°C und der in Tabelle 8 gezeigten Zusammensetzung.

**Tabelle 8**

| Komponente | Vol.-% |
|---|---|
| BUTAN | 0,009 |
| I-BUTAN | 0,002 |
| I-BUTEN | 0,001 |
| 1-BUTEN | 0,007 |
| C-2-BUTEN | 0,006 |
| T-2-BUTEN | 0,016 |
| 1.3-BUTADIEN | 0,004 |
| H2O | 0,84 |
| Sauerstoff | 5,79 |
| Stickstoff | 93,325 |

Der Absorptionsmittelstrom **5** wird auf 75°C erhitzt und tritt als Strom **6** auf der 30. Stufe der 36 Glockenböden umfassenden Desorptionskolonne in diese ein. Die Desorptionskolonne wird mit einem Kopfdruck von 5,5 bar absolut betrieben und es werden in ihr die C4-Kohlenwasserstoffe mit dem 156°C heißen Wasserdampfstrom **12** aus der Absorptionsmischung gestrippt. Das Gemisch aus Wasser und nicht mehr beladenem Absorptionsmittel verlässt den Sumpf der Desorptionskolonne als Strom **8** mit einer Temperatur von 157°C und besitzt die Zusammensetzung wie in Tabelle 9 aufgeführt.

**Tabelle 9**

| Komponente | Gewichts-% |
|---|---|
| BUTAN | 0,0 |
| I-BUTAN | 0,0 |
| I-BUTEN | 0,0 |
| 1-BUTEN | 0,0 |
| C-2-BUTEN | 0,0 |
| T-2-BUTEN | 0,0 |
| 1.3-BUTADIEN | 0,0 |
| H₂O | 8 |
| Mesitylen | 92 |
| Sauerstoff | 0,0 |
| Stickstoff | 0,0 |

Der Strom **8** wird anschließend auf 35°C abgekühlt, auf 10 bar absolut komprimiert und im Dekanter 10 in einen mesitylenreichen **11** und einen wässrigen Strom **12** getrennt.

Am Kopf der Desorptionskolonne werden die C₄-Komponenten im Strom **13** bei einer Temperatur von 45,8°C abgezogen und anschließend auf 13,8°C abgekühlt und damit teilweise kondensiert. Das Kondensat wird zum Teil in den Kopf der Desorptionskolonne zurückgeführt. Das Massenverhältnis zwischen den Strömen **14** und **16** ist hier 1 zu 1,05. Die Zusammensetzung des gasförmigen Stroms **15** und des flüssigen Stroms **16** sind in Tabelle 10 aufgeführt.

**Tabelle 10**

| Komponente | Strom 15 Gewichts-% | Strom 16 Gewichts-% |
|---|---|---|
| BUTAN | 7,4 | 17,7 |
| I-BUTAN | 2,6 | 4,6 |
| I-BUTEN | 0,01 | 0,03 |
| 1-BUTEN | 0,1 | 0,29 |
| C-2-BUTEN | 0,6 | 1,7 |
| T-2-BUTEN | 1,1 | 3,1 |
| 1.3-BUTADIEN | 28,7 | 72,28 |
| H2O | 0,7 | 0,3 |
| Mesitylen | 0,0 | 0,0 |
| Sauerstoff | 0,16 | 0,0 |
| Stickstoff | 58,63 | 0,0 |

Der Summe der Sauerstoffkonzentration in den Strömen 15 und 16 betrug in diesem Versuch 231 ppm. Es wurde also erfindungsgemäß eine signifikante Sauerstoffentfernung erzielt. Eine weitere Senkung des Sauerstoffgehalts ist durch die Erhöhung des Stickstoffstroms 2 erzielbar.

## Patentansprüche

1. Verfahren zur Herstellung von Butadien aus n-Butenen mit den Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, gegebenenfalls Kohlenstoffoxide und gegebenenfalls Inertgase erhalten wird;
C) Abkühlung und Kompression des Produktgasstroms b in mindestens einer Kompressionsstufe, wobei mindestens ein Kondensatstrom c1 enthaltend Wasser und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
D) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch
Da) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
Db) Entfernung von Sauerstoff aus dem mit C₄-Kohlenwasserstoffen beladenen Absorptionsmittelstrom durch Strippung mit einem Inertgas, und
Dc) Desorption der C₄-Kohlenwasserstoffe aus dem beladenen Absorptionsmittelstrom, wobei ein C₄-Produktgasstrom d1 erhalten wird, der weniger als 100 ppm Sauerstoff umfasst,
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2.

## Claims

1. A process for preparing butadiene from n-butenes, which comprises the following steps:
A) provision of a feed gas stream a comprising n-butenes;
B) introduction of the feed gas stream a comprising n-butenes and an oxygen-comprising gas into at least one dehydrogenation zone and oxidative dehydrogenation of n-butenes to butadiene, giving a product gas stream b comprising butadiene, unreacted n-butenes, water vapor, oxygen, low-boiling hydrocarbons, possibly carbon oxides and possibly inert gases;
C) cooling and compression of the product gas stream b in at least one compression stage, giving at least one condensate stream c1 comprising water and a gas stream c2 comprising butadiene, n-butenes, water vapor, oxygen, low-boiling hydrocarbons, possibly carbon oxides and possibly inert gases;
D) separation of incondensable and low-boiling gas constituents comprising oxygen, low-boiling hydrocarbons, possibly carbon oxides and possibly inert gases as gas stream d2 from the gas stream c2 by
Da) absorption of the C₄-hydrocarbons comprising butadiene and n-butenes in a high-boiling absorption medium, giving an absorption medium stream loaded with C₄-hydrocarbons and the gas stream d2,
Db) removal of oxygen from the absorption medium stream loaded with C₄-hydrocarbons by stripping with an inert gas and
Dc) desorption of the C₄-hydrocarbons from the loaded absorption medium stream to give a C₄ product gas stream d1 which comprises less than 100 ppm of oxygen;
E) separation of the C₄ product stream d1 by extractive distillation with a solvent which is selective for butadiene into a stream e1 comprising butadiene and the selective solvent and a stream e2 comprising n-butenes;
F) distillation of the stream e1 comprising butadiene and the selective solvent to give a stream f1 consisting essentially of the selective solvent and a butadiene-comprising stream f2.

## Revendications

1. Procédé pour la production de butadiène à partir de n-butènes, comportant les étapes :
A) disposition d'un courant de gaz de départ a contenant des n-butènes ;
B) introduction du courant de gaz de départ a contenant des n-butènes et d'un gaz contenant de l'oxygène dans au moins une zone de déshydrogénation et déshydrogénation oxydative de n-butènes en butadiène, avec obtention d'un courant de gaz produit b contenant du butadiène, des n-butènes n'ayant pas réagi, de la vapeur d'eau, de l'oxygène, des hydrocarbures à bas point d'ébullition, éventuellement des oxydes de carbone et éventuellement des gaz inertes ;
C) refroidissement et compression du courant de gaz produit b dans au moins un étage de compression, avec obtention d'au moins un courant de condensat c1 contenant de l'eau et un courant de gaz c2 contenant du butadiène, des n-butènes, de la vapeur d'eau, de l'oxygène, des hydrocarbures à bas point d'ébullition, éventuellement des oxydes de carbone et éventuellement des gaz inertes ;
D) à partir du courant de gaz c2, séparation de composants gazeux non condensables et à bas point d'ébullition, comprenant de l'oxygène, des hydrocarbures à bas point d'ébullition, éventuellement des oxydes de carbone et éventuellement des gaz inertes, en tant que courant de gaz d2, par
Da) absorption des hydrocarbures en C₄ comprenant le butadiène et les n-butènes dans un absorbant à haut point d'ébullition, avec obtention d'un courant d'absorbant chargé d'hydrocarbures en C₄ et du courant de gaz d2,
Db) élimination de l'oxygène à partir du courant d'absorbant chargé d'hydrocarbures en C₄, par strippage avec un gaz inerte, et
Dc) désorption des hydrocarbures en C₄ à partir du courant d'absorbant chargé, avec obtention d'un courant de gaz produit en C₄ d1, qui comprend moins de 100 ppm d'oxygène,
E) fractionnement du courant de produit en C₄ d1 par distillation extractive avec un solvant sélectif pour le butadiène, en un courant de substances e1 contenant du butadiène et le solvant sélectif et un courant de substances e2 contenant des n-butènes ;
F) distillation du courant de substances e1 contenant du butadiène et le solvant sélectif, en un courant de substances f1 consistant essentiellement en le solvant sélectif et un courant de substances f2 contenant du butadiène.
